# EUROPEAN PATENT APPLICATION

(11) **EP 4 487 845 A1**
(43) Date of publication of application: **08.01.2025**
(21) Application number: 23763716.0
(22) Date of filing: 02.03.2023
(51) Int. Cl.: A61K 31/216, A61P 27/02, A61P 27/04, A23L 33/10, A61K 9/00

(54) **COMPOSITION FOR PREVENTING, ALLEVIATING OR TREATING DRY EYE SYNDROME OR MEIBOMIAN GLAND DYSFUNCTION**

(30) Priority: 03.03.2022 KR 20220027751
(71) Applicant: Seoul National University Hospital, Seoul 03080 (KR)
(72) Inventor: OH, Joo Youn, Seoul 03080 (KR); YOON, Chang Ho, Seoul 03080 (KR); CHUNG, Jin Ho, Seoul 04428 (KR); OH, Jang-Hee, Seoul 03075 (KR); JANG, Hyun-Jae, Daejeon 34141 (KR); OH, Sei-Ryang, Daejeon 34141 (KR); AHN, Kyung Seop, Daejeon 34141 (KR)
(74) Representative: Dehns
(86) International application number: PCT/KR2023/002889
(87) International publication number: WO 2023/167518

(57) **Abstract**

The present invention relates to: a composition for preventing, alleviating or treating dry eye syndrome or meibomian gland dysfunction; and the like, and was completed by identifying the excellent effects of treating dry eye syndrome with 1,5-O-dicaffeoylquinic acid and improving meibomian gland function. 1,5-O-dicaffeoylquinic acid, a key component of the present invention, reduces corneal epithelial loss, increases the amount of tear production, inhibits inflammation of the lacrimal and meibomian glands, increases the area of the meibomian gland, increases the survival rate of corneal epithelial cells in a hyperosmotic environment, and can inhibit cell death. Therefore, the composition according to the present invention can effectively treat dry eye syndrome caused by environmental stress or various diseases and improve meibomian gland function, and thus is expected to be used as a therapeutic agent for various eye diseases.

## Description

### [Technical Field]

The present invention relates to a composition for preventing, alleviating, or treating dry eye syndrome and meibomian gland dysfunction.

This application claims priority to and the benefit of Korean Patent Application No. 10-2022-0027751, filed on March 03, 2022, the disclosure of which is incorporated herein by reference in its entirety.

### [Background Art]

Dry eye syndrome is a very common disease worldwide with a reported prevalence of 5.5% to 33.7% depending on the study population, gender, age, and diagnostic criteria. Dry eye syndrome causes symptoms such as dry eye, irritation, a foreign body sensation, pain, and a burning sensation, and is characterized by punctate epithelial erosion and irregularity of the corneal surface, fluctuating and blurred vision, and an increased risk of corneal ulcers. Chronic dry eye syndrome and keratoconjunctivitis sicca (KCS) caused by tear film instability are known to cause inflammation, as evidenced by an increase in tear inflammation-mediating chemokines and cytokines, immune activation and increased expression of an adhesion molecule (HLA-DR and intercellular adhesion molecule [ICAM-1]) by conjunctival epithelial cells, and an increased number of T lymphocytes in the conjunctiva. Corneal ulcers caused by KCS may cause impaired vision, permanent vision loss, and even ocular erosion. The concentration and activity of matrix metalloproteinase-9 (MMP-9) have been reported to be significantly increased both in tears and in the corneal epithelium of patients and mice with dry eye syndrome.

Recently, evidence has been consistently reported that inflammation and cell death on the ocular surface, caused by dry eye syndrome, are the primary causes of dry eye pathogenesis, resulting in a vicious cycle. It is also known that the increase in inflammatory cytokines, such as interleukin (IL)-1, tumor necrosis factor (TNF)-α, and transforming growth factor (TGF)-β, which are found in hyperosmotic stress and dry eye syndrome, is associated with an increased expression of MMP-9 in the corneal epithelium. Such a chronic inflammatory environment is partly responsible for the characteristic pathological degeneration of the conjunctival epithelium, such as squamous metaplasia and the loss of conjunctival goblet cells.

Dry eye syndrome is caused by tear film instability and largely divided into two types depending on the cause of tear film instability. The first type is the evaporative type of dry eye syndrome, which is mainly caused by meibomian gland dysfunction. In addition, it can also be caused by abnormal tear evaporation due to eyelid dysfunction, or abnormal tear evaporation due to an irregular ocular surface caused by an ocular surface disease. The second type is the aqueous-deficient type of dry eye syndrome, which is largely divided into Sjögren's syndrome, an autoimmune disease, and non-Sjögren's syndrome. Sjögren's syndrome is a systemic immune disease in which tear secretion is reduced and tear inflammation-related factors are increased due to autoimmunity against the lacrimal glands. As a result, inflammation of the ocular surface increases, the corneal epithelium is damaged, conjunctival goblet cells decrease, eyelid inflammation increases, and meibomian gland dysfunction also occurs. Non-Sjögren's syndrome is caused by a decrease in the tear-producing function of the lacrimal glands, the most common cause of which is aging. The evaporative type is more common than the aqueous-deficient type, and mixed type dry eye syndrome in which the two types are mixed can also occur.

Meanwhile, as described above, meibomian gland dysfunction is one of the most important causes of dry eye syndrome, and refers to a condition in which the outlets of meibomian glands are blocked or qualitative and/or quantitative changes in the meibomian glands themselves occur. Meibomian glands are sebaceous glands that are present in the upper and lower eyelids, and outlets are located along the edge of the eyelids and secrete oil on the ocular surface, thereby forming the lipid layer of the tear film. The tear film lipid layer prevents tear evaporation. As described above, dry eye syndrome can be caused by a decrease in tear secretion or excessive tear evaporation. Particularly, in the case of evaporative dry eye syndrome caused by excessive tear evaporation, only about 14.5% of cases are due to a simple lack of tears, and many more cases are due to a decrease in the lipid layer of the eye caused by meibomian gland dysfunction. When oil is not secreted properly from the meibomian glands, the eyes may become dry and irritated, but on the other hand, when the concentration of oil becomes excessive, inflammation may occur, which can lead to serious diseases such as corneal ulcers.

Sjögren's syndrome is a systemic chronic inflammatory disease affecting the lacrimal glands and the salivary glands and is the second most common autoimmune disease following rheumatoid arthritis. Sjögren's syndrome is the second most common autoimmune disease, affecting 1 to 3% of the world's population, including 4,000,000 patients in the United States. 90% or more of patients with Sjögren's syndrome are female, and Sjögren's syndrome may be accompanied by various other autoimmune phenomena. Although the physiological cause of Sjögren's syndrome is not precisely understood, it is reported that two distinct phenomena occur. There are two distinct stages: the initial stage when the activity of autoreactive lymphatic cells is induced and the effect stage when pathogenic lymphatic cells infiltrate the lacrimal and salivary glands and reduce the amounts of tears and saliva, respectively. However, there is still no fundamental therapy for treating Sjögren's syndrome.

Recently, as the use of electronic devices such as computers and smartphones, or contact lenses increases, and environmental pollution such as dryness or fine dust also increases, the number of patients with dry eye syndrome or meibomian gland dysfunction is continuously increasing. Nevertheless, there is still no fundamental therapy for dry eye syndrome and/or meibomian gland dysfunction. Among the currently used treatments for eye diseases, steroid preparations have an inflammation inhibitory effect, but also have the side effects of worsening corneal epithelial defects and delaying wound healing, and artificial tears, which are commonly used by patients with dry eye syndrome, have minimal effect on wound healing and no effect on inhibiting inflammation. Therefore, there is an urgent need to discover treatments that can effectively prevent, alleviate, or treat dry eye syndrome and/or meibomian gland dysfunction.

### [Disclosure]

### [Technical Problem]

The present invention was conceived to solve the above problems, and it was completed by confirming that 1,5-O-dicaffeoylquinic acid has an excellent effect on preventing and treating dry eye syndrome, and can alleviate and treat meibomian gland dysfunction.

Therefore, the present invention, which includes 1,5-O-dicaffeoylquinic acid, an isomer thereof, a solvate thereof, or a pharmaceutically acceptable salt thereof as an active ingredient, is directed to providing a composition for preventing, alleviating, and/or treating dry eye syndrome or meibomian gland dysfunction.

However, technical problems to be solved by the present invention are not just limited to the above-described problems, but also cover other problems which are not described herein, which will be fully understood by those of ordinary skill in the art from the following descriptions.

### [Technical Solution]

The present invention provides a pharmaceutical composition for preventing or treating dry eye syndrome and/or meibomian gland dysfunction, which includes 1,5-0-dicaffeoylquinic acid, an isomer thereof, a solvate thereof, or a pharmaceutically acceptable salt thereof as an active ingredient.

In one embodiment of the present invention, the dry eye syndrome and/or meibomian gland dysfunction may be caused by one or more selected from the group consisting of Sjögren's syndrome, styes, blepharitis, a dry environment, keratoconjunctival epithelial disorder, corneal xerosis, alacrima, ocular graft-versus-host disease, Stevens-Johnson syndrome, ocular pemphigoid, drug-induced dry eye syndrome, dry eye syndrome after ophthalmic surgery, dry eye syndrome with allergic conjunctivitis, visual display terminal (VDT) syndrome, and contact lens wear, but the present invention is not limited thereto.

In another embodiment of the present invention, the composition may be a formulation for oral administration or an eye drop formulation, but the present invention is not limited thereto.

In still another embodiment of the present invention, the composition may increase the amount of tear secretion from the lacrimal glands, but the present invention is not limited thereto.

In yet another embodiment of the present invention, the composition may decrease corneal epithelial defects, but the present invention is not limited thereto.

In yet another embodiment of the present invention, the composition may satisfy one or more characteristics selected from the group consisting of the following, but the present invention is not limited thereto:
(a) a decrease in the amount or activity of IL-1β in the lacrimal glands; and
(b) a decrease in the infiltration of CD3 positive immune cells in the lacrimal glands.

In yet another embodiment of the present invention, the composition may satisfy one or more characteristics selected from the group consisting of the following, but the present invention is not limited thereto:
an increase in the amount of tear secretion from the lacrimal glands;
a decrease in corneal epithelial defects;
a decrease in the expression level or activity of IL-1β in the lacrimal glands; and
a decrease in the infiltration of CD3 positive immune cells in the lacrimal glands.

In yet another embodiment of the present invention, the composition may satisfy one or more characteristics selected from the group consisting of the following, but the present invention is not limited thereto:
(a) a decrease in the expression level or activity of one or more selected from the group consisting of IL-6, TNFα, and MMP9 in the meibomian glands;
(b) an increase in the area of the meibomian glands; and
(c) an increase in the expression level or activity of meibomian gland differentiation factors in the eyelids.

In yet another embodiment of the present invention, the composition may satisfy one or more characteristics selected from the group consisting of the following, but the present invention is not limited thereto:
(a) increased survival of corneal epithelial cells in dry eye syndrome or similar stress environments; and
(b) decreased apoptosis of corneal epithelial cells in dry eye syndrome or similar stress environments.

The present invention also provides an ophthalmic kit, which includes the composition according to the present invention. The ophthalmic kit includes a kit for preventing or treating dry eye syndrome and meibomian gland dysfunction. That is, the present invention provides a kit for preventing or treating dry eye syndrome or meibomian gland dysfunction, which includes the composition.

The present invention also provides a food composition for preventing or alleviating dry eye syndrome and meibomian gland dysfunction, which includes 1,5-O-dicaffeoylquinic acid, an isomer thereof, a solvate thereof, or a pharmaceutically acceptable salt thereof as an active ingredient. The food composition includes a health functional food composition.

The present invention also provides an ophthalmic composition, which includes 1,5-O-dicaffeoylquinic acid, an isomer thereof, a solvate thereof, or a pharmaceutically acceptable salt thereof as an active ingredient.

The present invention also provides a method of treating dry eye syndrome or meibomian gland dysfunction, which includes administering a composition including 1,5-O-dicaffeoylquinic acid, an isomer thereof, a solvate thereof, or a pharmaceutically acceptable salt thereof as an active ingredient to a subject in need thereof.

The present invention also provides a use of a composition including 1,5-0-dicaffeoylquinic acid, an isomer thereof, a solvate thereof, or a pharmaceutically acceptable salt thereof as an active ingredient for preventing, alleviating, or treating dry eye syndrome or meibomian gland dysfunction.

In addition, the present invention provides a use of a composition including 1,5-0-dicaffeoylquinic acid, an isomer thereof, a solvate thereof, or a pharmaceutically acceptable salt thereof as an active ingredient for preparing a drug for treating dry eye syndrome or meibomian gland dysfunction.

### [Advantageous Effects]

The present invention relates to a composition for preventing, alleviating, or treating dry eye syndrome or meibomian gland dysfunction, which was completed by confirming an excellent effect of 1,5-O-dicaffeoylquinic acid for treating dry eye syndrome and improving meibomian gland function. The key component of the present invention, 1,5-0-dicaffeoylquinic acid, can not only reduce corneal epithelial defects and increase the amount of tear secretion, but can also inhibit inflammation of the lacrimal glands and the meibomian glands, increase the area of the meibomian glands, increase the viability of corneal epithelial cells in a hyperosmotic environment, and inhibit apoptosis. Therefore, the composition according to the present invention can effectively treat dry eye syndrome due to environmental stress or various diseases and improve meibomian gland function, so it is expected to be used in various treatments for eye diseases.

### [Description of Drawings]

FIG. 1 shows the results of confirming the degree of corneal epithelial defects and the amount of tear secretion by staining dry eye syndrome mouse models undergoing desiccating stress with Lissamine green vital dye after instilling 1,5-O-dicaffeoylquinic acid (1,5DQ) or PBS (phosphate-buffered solution) into their eyes.
FIG. 2 shows the results of confirming the degree of corneal epithelial defects and the amount of tear secretion by staining Sjögren's syndrome-related inflammatory dry eye syndrome mouse models with Lissamine green vital dye after instilling 1,5DQ or PBS into their eyes.
FIGS. 3A and 3B show the results of measuring the expression levels of interleukin 1β in the extraorbital lacrimal gland (FIG. 3A) and the intraorbital lacrimal gland (FIG. 3B) after instilling 1,5DQ or PBS into the eyes of dry eye syndrome mouse models undergoing desiccating stress.
FIGS. 4A to 4C show the results of observing CD3-positive immune cells using a microscope (bar length = 200 µm) and quantifying the degree of infiltration of immune cells in the lacrimal glands by immunohistochemical staining with CD3 in the extraorbital lacrimal gland (FIG. 4A) and the intraorbital lacrimal gland (FIG. 4B) after instilling 1,5DQ or PBS into the eyes of Sjögren's syndrome-related inflammatory dry eye syndrome mouse models (FIG. 4C).
FIG. 5 shows the results of measuring the expression levels of IL-6, TNFα, and MMP9 in the eyelids where the meibomian glands are located after instilling 1,5DQ or PBS into the eyes of Sjögren's syndrome-related inflammatory dry eye syndrome mouse models.
FIGS. 6A and 6B show the results of observing meibomian glands of the eyelids (FIG. 6A; the horizontal length of each image of the meibomian glands =3 mm, the bar length of a fluorescence image = 100 µm) and the results of measuring the area of the meibomian glands (FIG. 6B) after instilling 1,5DQ or PBS into the eyes of Sjögren's syndrome-related inflammatory dry eye syndrome mouse models.
FIG. 7 shows the results of measuring the expression levels of meibomian gland differentiation factors of the eyelids after instilling 1,5DQ or PBS into the eyes of dry eye syndrome mouse models undergoing desiccating stress.
FIG. 8A shows the results of observing corneal epithelial defects by staining the cornea with Lissamine green vital dye after instilling PBS or various concentrations of 1,5DQ into the eyes of Sjögren's syndrome-related inflammatory dry eye syndrome mouse models.
FIG. 8B shows the results of measuring the degree of corneal epithelial defects after instilling PBS or various concentrations of 1,5DQ into the eyes of Sjögren's syndrome-related inflammatory dry eye syndrome mouse models.
FIG. 8C shows the results of measuring the amount of tear secretion after instilling PBS or various concentrations of 1,5DQ into the eyes of Sjögren's syndrome-related inflammatory dry eye syndrome mouse models.
FIG. 9A shows the result of measuring cell viability after treating human corneal epithelial cells with various concentrations of 1,5DQ under a normal environment.
FIG. 9B shows the result of measuring cell viability after treating human corneal epithelial cells with various concentrations of 1,5DQ under a hyperosmotic environment (100 mM NaCl treatment).
FIG. 10 shows the result of measuring the degree of apoptosis after treating human corneal epithelial cells with various concentrations of 1,5DQ under a hyperosmotic environment (100 mM NaCl treatment).

### [Best Mode]

The present invention relates to a composition for preventing, alleviating, or treating dry eye syndrome or meibomian gland dysfunction, which was completed by confirming that 1,5-O-dicaffeoylquinic acid (1,5DQ) has an excellent effect on preventing and treating dry eye syndrome and is capable of alleviating and treating meibomian gland dysfunction.

Specifically, in one embodiment of the present invention, as a result of instilling 1,5DQ into the eyes of two types of dry eye syndrome (desiccating stress-induced dry eye syndrome and Sjögren's syndrome-related dry eye syndrome) mouse models, it was confirmed that the two mouse models show reduced corneal epithelial defects and an increased amount of tear secretion (Example 1).

In another embodiment of the present invention, as a result of instilling 1,5DQ into the eyes of the eye syndrome mouse models, the level of inflammatory cytokines decreased and the infiltration of inflammatory cells decreased in the lacrimal glands, confirming that 1,5DQ is effective in suppressing inflammation in the lacrimal glands (Example 2).

In still another embodiment of the present invention, as a result of instilling 1,5DQ into the eyes of dry eye syndrome mouse models, the level of inflammatory cytokines in the meibomian glands decreased, the area of the meibomian glands increased, the transcription of meibomian gland differentiation factors in the eyelids increased, confirming that 1,5DQ is effective in suppressing inflammation in the meibomian glands and improving meibomian gland function (Example 3).

In yet another embodiment of the present invention, as a result of instilling various concentrations of 1,5DQ into the eyes of the eye syndrome mouse models, it was confirmed that the effect of treating dry eye syndrome by 1,5DQ is dependent on the concentration of 1,5DQ (Example 4).

In yet another embodiment of the present invention, as a result of treating human corneal epithelial cells with 1,5DQ while exposing them to a hyperosmotic environment, the survival of corneal epithelial cells increased and apoptosis decreased, confirming that 1,5DQ can protect corneal epithelial cells in a hyperosmotic environment (Example 5).

The embodiments support that 1,5-O-dicaffeoylquinic acid can improve the function of the lacrimal and meibomian glands and exhibit an excellent therapeutic effect on dry eye syndrome and meibomian gland dysfunction.

Hereinafter, the present invention will be described in detail.

The present invention provides a pharmaceutical composition for preventing or treating an eye disease, which includes 1,5-O-dicaffeoylquinic acid, an isomer thereof, a solvate thereof, or a pharmaceutically acceptable salt thereof as an active ingredient.

In the present invention, 1,5-O-dicaffeoylquinic acid (1,5DQ) is a polyphenol compound first isolated from coffee beans, is characterized in that two caffeic acids are connected to one quinic acid molecule by ester bonds. 1,5DQ may inhibit HIV-1 replication, and it has been reported to have a modified prion suppression effect (Korean Patent No. 10-1852570), but nothing is known about its effect in treating an eye disease. The 1,5-O-dicaffeoylquinic acid according to the present invention may be represented by any one of Chemical Formulas 1 to 3 below, but the present invention is not limited thereto.

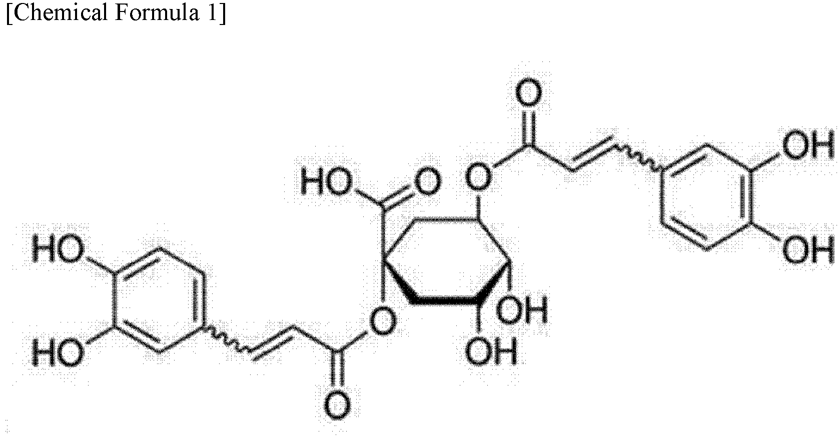

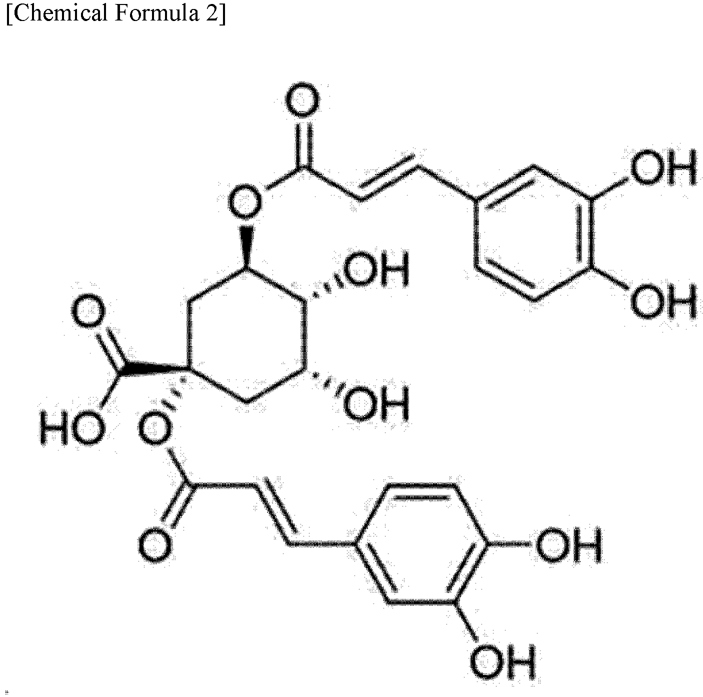

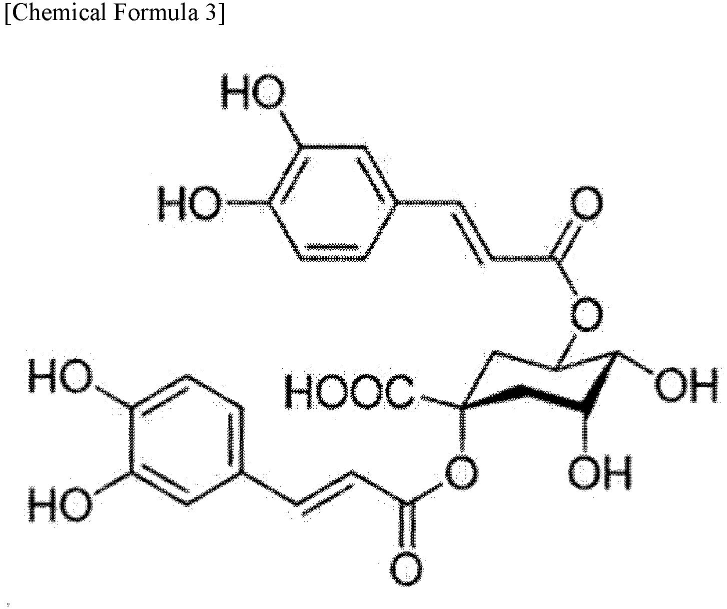

The scope of the compound of the present invention may include not only a pharmaceutically acceptable salt but also all isomers, hydrates and solvates, which can be prepared by conventional methods.

Specifically, the compounds according to the present invention may have nonaromatic double bonds and one or more asymmetric centers. Therefore, they can be generated as racemates and racemic mixtures, single enantiomers, stereoisomers, individual diastereomers, diastereomeric mixtures, and cis- or trans-isomers. All of these isomeric forms are contemplated. That is, the present invention may include an isomer of 1,5-0-dicaffeoylquinic acid as an active ingredient.

In addition, the present invention may include a solvate of 1,5-O-dicaffeoylquinic acid as an active ingredient. The term "solvate" used herein refers to a compound solvated in an organic or inorganic solvent. The solvate is, for example, a hydrate.

In addition, the present invention may include a pharmaceutically acceptable salt of 1,5-O-dicaffeoylquinic acid. The term "pharmaceutically acceptable salt" used herein includes salts induced from pharmaceutically acceptable inorganic acids, organic acids, or bases.

The term "pharmaceutically acceptable" used herein refers to a compound or composition which is suitable for use in contact with tissue of a subject (e.g., a human) due to a reasonable benefit/risk ratio without excessive toxicity, irritation, allergic responses or other problems or complications, and is included in the scope of sound medical judgment.

Examples of suitable acids may include hydrochloric acid, hydrobromic acid, sulfuric acid, nitric acid, perchloric acid, fumaric acid, maleic acid, phosphoric acid, glycolic acid, lactic acid, salicylic acid, succinic acid, p-toluenesulfonic acid, tartaric acid, acetic acid, citric acid, methanesulfonic acid, formic acid, benzoic acid, malonic acid, gluconic acid, naphthalene-2-sulfonic acid, and benzenesulfonic acid. The acid addition salt may be prepared by a conventional method, for example, by dissolving a compound in an excessive amount of acid aqueous solution, and precipitating the salt using a water-miscible organic solvent such as methanol, ethanol, acetone or acetonitrile. Alternatively, the acid addition salt may be prepared by heating equimolar amounts of compound and an acid or alcohol in water, and evaporating or drying the mixture, or suction filtering the precipitated salt.

Salts derived from suitable bases may include alkali metals such as sodium and potassium, alkaline earth metals such as magnesium, and ammonium, but the present invention is not limited thereto. The alkali metals or alkaline earth metals may be obtained by, for example, dissolving a compound in an excessive alkali metal hydroxide or alkaline earth metal hydroxide solution, filtering an undissolved compound salt, and evaporating and drying the filtrate. Here, it is particularly pharmaceutically suitable to prepare a sodium, potassium or calcium salt as a metal salt, and a corresponding silver salt may be obtained by reacting an alkali metal or alkaline earth metal salt with a suitable silver salt (e.g., silver nitrate).

The "eye disease" used herein refers to an illness, condition, and/or a disease affecting or relating to an eye or a part or area of the eye, and in a broad sense, the eye includes the eyeball, tissue and fluids that constitute the eyeball, extraocular muscles (oblique and rectus muscles), and the portion of the optic nerve in or adjacent to the eyeball.

In the present invention, the eye disease may be, but is not limited to, for example, one or more selected from the group consisting of uveitis, dry eye syndrome, meibomian gland dysfunction, blepharitis, styes, Sjögren's syndrome, Stevens-Johnson syndrome, ocular graft-versus-host disease, pemphigoid, diabetic retinopathy, diabetic macular edema, age-related macular degeneration, retinopathy of prematurity, polypoidal choroidal vasculopathy, ischemic proliferative retinopathy, retinitis pigmentosa, cone dystrophy, proliferative vitreoretinopathy, retinal artery occlusion, retinal vein occlusion, pterygium, retinitis, keratitis, persistent corneal epithelial defects, recurrent corneal erosion, exposure keratopathy, neurotrophic keratopathy, ocular surface burns, conjunctivitis, Leber's optic atrophy, retinal detachment, retinal pigment epithelium detachment, neovascular glaucoma, corneal neovascularization, retinal neovascularization, and choroidal neovascularization. In addition, the eye disease of the present invention may include ocular side effects resulting from the use of contact lens, and may specifically be accompanied by eye discomfort, dryness, burning, stinging, and non-bacterial inflammation, but the present invention is not limited thereto.

Preferably, the eye disease is dry eye syndrome or meibomian gland dysfunction.

In the present invention, meibomian gland dysfunction is a chronic disease that occurs in the meibomian glands which are present in the eyelids and have outlets along the edge of the eyelids, and includes cases where the outlets of the meibomian glands are blocked or the meibomian glands themselves undergo qualitative or quantitative changes (particularly, a decrease in area of the meibomian glands). The meibomian glands contribute to the formation of a lipid layer within the tear layer, and the lipid layer delays the evaporation of tears, reduces the surface tension of tears, and aids in the absorption of water into the tear layer. As a result, the tear layer is maintained thicker. In addition, it closes the space between the eyelids during sleep, provides lubrication on the ocular surface, and increases the spread and stability of the tear layer. Therefore, even when tear production is normal, when there is an abnormality in the lipid layer of tears, tear evaporation increases, resulting in not only inflammation due to increased osmotic pressure but also dry eye syndrome. Preferably, the meibomian gland dysfunction according to the present invention may be caused by inflammation of the meibomian glands and a decrease in the area of the meibomian glands, but the present invention is not limited thereto.

In the present invention, dry eye syndrome (dry eye disease) refers to a disease that is accompanied by various ocular symptoms due to the loss of tear film homeostasis due to causes such as the instability of the tear film, the high osmotic pressure of tears, the damage and inflammation of the ocular surface, and abnormalities in the sensory nerves. Specifically, dry eye syndrome is characterized by damage to the ocular surface, the occurrence of irritating symptoms such as soreness, dryness, irritation, a foreign body sensation, pain, and a burning sensation in the eyes due to the lack of tears, excessive tear evaporation, or an unbalanced tear composition. Dry eye syndrome involves disorders of the tear film due to tear deficiency or excessive evaporation and may also include symptoms of eye discomfort that cause damage to the interpalpebral ocular surface. In addition, dry eye syndrome may refer to a multifactorial disease of tears and the ocular surface, which causes potential damage to the ocular surface along with symptoms of discomfort, visual impairment and tear film instability, and this disease may be accompanied by increased osmotic pressure of the tear film and inflammation on the ocular surface. The inflammation of the tear producing organ can cause dry eye syndrome, and systemic diseases such as Sjögren's syndrome, Stevens-Johnson syndrome, and pemphigoid can also cause dry eye syndrome. In addition, dry eye syndrome includes aqueous-deficient dry eye syndrome and evaporative dry eye syndrome, wherein the aqueous-deficient dry eye syndrome may include conditions caused by insufficient tear secretion from the lacrimal glands. In addition, the evaporative dry eye syndrome may include conditions caused by excessive water loss from the exposed ocular surface in the presence of the normal secretory function of the lacrimal glands. Preferably, the dry eye syndrome according to the present invention may be due to inflammation of the lacrimal glands, denervation of the cornea, meibomian gland dysfunction, eyelid dysfunction, and keratoconjunctival epithelial disorders.

Most preferably, the dry eye syndrome or meibomian gland dysfunction is caused by one or more selected from the group consisting of Sjögren's syndrome, styes, blepharitis, a dry environment, keratoconjunctival epithelial disorder, corneal xerosis, alacrima, ocular graft-versus-host disease, Stevens-Johnson syndrome, ocular pemphigoid, drug-induced dry eye syndrome, dry eye syndrome after ophthalmic surgery, dry eye syndrome with allergic conjunctivitis, visual display terminal (VDT) syndrome, and contact lens wear.

The "keratoconjunctival epithelial disorders" used herein may include dry eye, corneal epithelial defects, conjunctival epithelial defects, corneal epithelial erosion, decreased corneal thickness, corneal infiltration, corneal perforations or corneal epithelial desquamation; corneal ulcers, keratitis, conjunctivitis, superficial punctate keratitis, keratoconjunctivitis sicca (KCS), superior limbic keratoconjunctivitis, filamentary keratitis, keratomycosis, infectious eye diseases of the keratoconjunctival epithelium, intraocular trauma, pemphigoid, microsurgery, or keratoconjunctival epithelial disorders associated with wearing hard contact lens.

The composition according to the present invention may be formulated into various forms according to conventional methods and used. For example, the composition according to the present invention may be prepared in the form of a solution, a suspension, a syrup, an emulsion, a liposome, a powder, a granule, a tablet, a sustained-release preparation, an eye drop, a capsule, a contact lens cleaner, or a contact lens lubricant. Preferably, the composition according to the present invention may be prepared in a formulation for oral administration or eye drops.

The composition according to the present invention may elevate the amount of tear secretion from the lacrimal glands, and/or reduce corneal epithelial defects. The corneal epithelial defects include persistent corneal epithelial defects. The corneal epithelial defects may be due to inflammation, chemical burns, a denervated cornea, tear deficiency, and radiation keratitis. In addition, the composition according to the present invention may suppress inflammation of the lacrimal glands and/or the meibomian glands.

In addition, the composition according to the present invention may satisfy one or more characteristics selected from the group consisting of the following:
(a) a decrease in the expression level or activity of inflammatory cytokines in the lacrimal glands; and
(b) a decrease in the infiltration of inflammatory immune cells in the lacrimal glands.

In one exemplary embodiment of the present invention, the inflammatory cytokine may be interleukin 1β (IL-1β), but the present invention is not limited thereto. In another exemplary embodiment of the present invention, the inflammatory immune cells are preferably CD3-positive cells, and more preferably CD3-positive T cells, but the present invention is not limited thereto.

In addition, the composition according to the present invention may satisfy one or more characteristics selected from the group consisting of the following:
(a) a decrease in the expression level or activity of inflammatory cytokines in the meibomian glands;
(b) an increase in the area of the meibomian glands; and
(c) an increase in the expression level or activity of meibomian gland differentiation factors in the eyelids.

In one embodiment of the present invention, the inflammatory cytokine may be one or more selected from the group consisting of IL-6, TNFα, and MMP9, but the present invention is not limited thereto. In another embodiment of the present invention, the meibomian gland differentiation factor may be one or more selected from the group consisting of SCD1 and/or SCD3, but the present invention is not limited thereto.

In addition, the composition according to the present invention may satisfy one or more characteristics selected from the group consisting of the following:
(a) increased survival of corneal epithelial cells in dry eye syndrome or similar stress environments; and
(b) decreased apoptosis of corneal epithelial cells in dry eye syndrome or similar stress environments.

The similar stress environment means a stress environment that can cause eye diseases such as dry eye syndrome or meibomian gland dysfunction. For example, the similar stress environment includes a dry environment, a hyperosmotic environment, and an inflammatory environment, and may include any kind of environment that can induce abnormal tear evaporation, an unstable tear film, and/or an abnormal decrease in the amount of tear secretion.

Preferably, the composition according to the present invention may increase the survival or division ability of corneal epithelial cells under a hyperosmotic environment and/or reduce the apoptosis of corneal epithelial cells.

The content of the 1,5-O-dicaffeoylquinic acid or a pharmaceutically acceptable salt thereof in the composition of the present invention can be appropriately adjusted depending on the symptoms of a disease, the degree of progression of the symptoms, and a patient's condition, and for example, the content may be 0.0001 to 99.9 wt%, or 0.001 to 50 wt% based on the total weight of the composition, but the present invention is not limited thereto. The content is a value based on the dry weight in which a solvent is removed.

In addition, in the present invention, the 1,5-O-dicaffeoylquinic acid or a pharmaceutically acceptable salt thereof maybe included at a concentration of 1 µg/mL to 10 mg/mL, 1 µg/mL to 9 mg/mL, 1 µg/mL to 8 mg/mL, 1 µg/mL to 7 mg/mL, 1 µg/mL to 6 mg/mL, 1 µg/mL to 5 mg/mL, 1 µg/mL to 4 mg/mL, 1 µg/mL to 3 mg/mL, 1 µg/mL to 2 mg/mL, 1 µg/mL to 1 mg/mL, 1 µg/mL to 900 µg/mL, 1 µg/mL to 700 µg/mL, 1 µg/mL to 500 µg/mL, 1 µg/mL to 300 µg/mL, 1 µg/mL to 100 µg/mL, 1 µg/mL to 90 µg/mL, 1 µg/mL to 70 µg/mL, 1 µg/mL to 50 µg/mL, 1 µg/mL to 30 µg/mL, 1 µg/mL to 20 µg/mL, or 1 µg/mL to 10 µg/mL with respect to the total composition, but the present invention is not limited thereto.

The pharmaceutical composition according to the present invention may further include appropriate carriers, excipients, and diluents, which are generally used in the preparation of pharmaceutical compositions. The excipient may be, for example, one or more selected from the group consisting of a diluent, a binder, a disintegrant, a lubricant, an adsorbent, a humectant, a film-coating material, and a controlled-release additive.

The pharmaceutical composition according to the present invention may be formulated in the form of a powder, a granule, a sustained-release granule, an enteric granule, a liquid, an ophthalmic solution, an elixir, an emulsion, a suspension, a spirit, a troche, aromatic water, a lemonade, a tablet, a sustained-release tablet, an enteric tablet, a sublingual tablet, a hard capsule, a soft capsule, a sustained-release capsule, an enteric capsule, a pill, a tincture, a soft extract, a dry extract, a fluid extract, an injection, a capsule, a perfusate, a plaster, a lotion, a paste, a spray, an inhalant, a patch, a sterile injection, or an external preparation such as an aerosol according to a conventional method, and the external preparation may be formulated as a cream, a gel, a patch, a spray, an ointment, a plaster, a lotion, a liniment, a paste or a cataplasma.

Carriers, excipients, and diluents, which can be included in the pharmaceutical composition according to the present invention, may include lactose, dextrose, sucrose, an oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil.

The composition according to the present invention may be prepared using a diluent or an excipient such as a filler, an extender, a binder, a wetting agent, a disintegrant, and a surfactant, which are commonly used.

Additives for a tablet, powder, granule, capsule, pill and troche may include excipients such as corn starch, potato starch, wheat starch, lactose, sucrose, glucose, fructose, di-mannitol, precipitated calcium carbonate, synthetic aluminum silicate, calcium monohydrogen phosphate, calcium sulfate, sodium chloride, sodium bicarbonate, purified lanolin, microcrystalline cellulose, dextrin, sodium alginate, methylcellulose, carboxymethylcellulose, sodium carboxymethylcellulose, kaolin, urea, colloidal silica gel, hydroxypropyl starch, hydroxypropyl methyl cellulose (HPMC) 1928, HPMC 2208, HPMC 2906, HPMC 2910, propylene glycol, casein, calcium lactate and Primojel; binders such as gelatin, gum arabic, ethanol, agar powder, cellulose acetate phthalate, carboxymethyl cellulose, carboxymethyl cellulose calcium, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch powder, hydroxypropylcellulose, hydroxypropylmethylcellulose, polyvinyl alcohol and polyvinylpyrrolidone; disintegrants such as hydroxypropylmethylcellulose, corn starch, agar powder, methylcellulose, bentonite, hydroxypropyl starch, sodium carboxymethylcellulose, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, sucrose, magnesium aluminum silicate, a di-sorbitol solution and light anhydrous silicic acid; and lubricants such as calcium stearate, magnesium stearate, stearic acid, hydrogenated vegetable oil, talc, lycopodium powder, kaolin, petrolatum, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, a higher fatty acid, a higher alcohol, silicone oil, paraffin oil, polyethylene glycol fatty acid ether, starch, sodium chloride, sodium acetate, sodium oleate, dl-leucine and light anhydrous silicic acid.

Additives for liquid formulations according to the present invention may be water, diluted hydrochloric acid, diluted sulfuric acid, sodium citrate, monostearate sucrose, polyoxyethylene sorbitol fatty acid esters (Tween esters), polyoxyethylene monoalkylethers, lanolin ethers, lanolin esters, acetic acid, hydrochloric acid, acetic acid, hydrochloric acid, aqueous ammonia, ammonium carbonate, potassium hydroxide, sodium hydroxide, prolamine, polyvinylpyrrolidone, ethyl cellulose, and sodium carboxymethylcellulose.

A syrup according to the present invention may include a solution of white sugar, a different type of sugar, or a sweetener, and if necessary, a flavoring agent, a colorant, a preservative, a stabilizer, a suspending agent, an emulsifier, or a thickener.

For an emulsion according to the present invention, distilled water may be used, and if necessary, an emulsifier, a preservative, a stabilizer, and a flavoring agent may be used.

A suspension according to the present invention may include a suspending agent such as acacia, tragacanth, methylcellulose, carboxymethylcellulose, sodium carboxymethylcellulose, microcrystalline cellulose, sodium alginate, hydroxypropylmethyl cellulose (HPMC), HPMC 1828, HPMC 2906, or HPMC 2910, and if necessary, a surfactant, a preservative, a stabilizer, a colorant, or a flavoring agent.

Injections according to the present invention may include a solvent such as injectable sterile water, 0.9% sodium chloride for injection, Ringer's solution, dextrose for injection, dextrose+sodium chloride for injection, PEG, lactated Ringer's solution, ethanol, propylene glycol, non-volatile oil-sesame oil, cottonseed oil, peanut oil, soybean oil, corn oil, ethyl oleate, isopropyl myristic acid or benzene benzoate; a solubilizing agent such as sodium benzoate, sodium salicylate, sodium acetate, urea, urethane, monoethylacetamine, butazolidine, propylene glycol, Tween, nicotinamide, hexamine or dimethylacetamide; a buffer such as a weak acid and a salt thereof (acetic acid and sodium acetate), a weak base and a salt thereof (ammonia and ammonium acetate), an organic compound, a protein, albumin, peptone, or gums; an isotonic agent such as sodium chloride; a stabilizer such as sodium bisulfite (NaHSO₃), carbon dioxide gas, sodium metabisulfite (Na₂S₂O₃), sodium sulfite (Na₂SO₃), nitrogen gas (N₂) or ethylenediaminetetracetic acid; an antioxidant such as sodium bisulfide 0.1%, sodium formaldehyde sulfoxylate, thiourea, disodium ethylenediaminetetraacetate or acetone sodium bisulfite; an analgesic such as benzyl alcohol, chlorobutanol, procaine hydrochloride, glucose or calcium gluconate; or a suspending agent such as sodium CMC, sodium alginate, Tween 80 or aluminum monostearate.

Suppositories according to the present invention may include a base such as cacao butter, lanolin, Witepsol, polyethylene glycol, glycerogelatin, methyl cellulose, carboxymethylcellulose, a mixture of stearate and oleate, Subanal, cottonseed oil, peanut oil, palm oil, cacao butter + cholesterol, lecithin, Lanette wax, glycerol monostearate, Tween or Span, Imhausen, monolene (propylene glycol monostearate), glycerin, Adeps solidus, Buytyrum Tego-G, Cebes Pharma 16, hexalide base 95, Cotomar, Hydrokote SP, S-70-XXA, S-70-XX75 (S-70-XX95), Hydrokote 25, Hydrokote 711, Idropostal, Massa estrarium, (A, AS, B, C, D, E, I, T), Mass-MF, Masupol, Masupol-15, neosuppostal-N, paramount-B, supposiro (OSI, OSIX, A, B, C, D, H, L), suppository base IV types (AB, B, A, BC, BBG, E, BGF, C, D, 299), Suppostal (N, Es), Wecoby (W, R, S, M ,Fs), or a Tegester triglyeride base (TG-95, MA, 57).

Solid preparations for oral administration include tablets, pills, powders, granules, and capsules, and such solid preparations are prepared by mixing at least one excipient, such as starch, calcium carbonate, sucrose, lactose, or gelatin with the composition. Aside from simple excipients, lubricants such as magnesium stearate and talc are further used.

A liquid formulation for oral administration may be a suspension, a liquid for internal use, an emulsion or a syrup, and a generally-used simple diluent such as water or liquid paraffin, as well as various types of excipients, for example, a wetting agent, a sweetener, a flavoring agent and a preservative may be included. Formulations for parenteral administration may include sterilized aqueous solutions, non-aqueous solvents, suspensions, emulsions, freeze-dried formulations, or suppositories. As the non-aqueous solvent or suspension, propylene glycol, polyethylene glycol, a vegetable oil such as olive oil, or an injectable ester such as ethyl oleate may be used.

The pharmaceutical composition of the present invention is administered at a pharmaceutically effective amount. The "pharmaceutically effective amount" used herein refers to an amount sufficient for treating a disease at a reasonable benefit/risk ratio applicable for medical treatment, and an effective dosage may be determined by parameters including the type of a patient's disease, severity, drug activity, sensitivity to a drug, administration time, an administration route and an excretion rate, the duration of treatment and drugs simultaneously used, and other parameters well known in the medical field.

The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without a side effect, and such a dose may be easily determined by one of ordinary skill in the art.

The pharmaceutical composition of the present invention may be administered to a subject via various routes. All administration routes may be contemplated, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention is determined according to the type of drug as an active ingredient as well as various related parameters such as a disease to be treated, an administration route, a patient's age, sex, body weight, and the severity of a disease.

The "subject" used herein refers to a subject in need of treatment for a disease, and more specifically, a mammal such as a human or a non-human primate, a mouse, a rat, a dog, a cat, a horse, or a cow.

The "administration" used herein refers to providing the given composition of the present invention to a subject by any suitable method.

The term "prevention" used herein refers to all actions that inhibit or delay the onset of a target disease, the term "treatment" used herein refers to all actions that alleviate or beneficially change a target disease and associated metabolic abnormalities thereof by administration of the pharmaceutical composition according to the present invention, and the term "alleviation" used herein refers to all types of actions that reduce parameters related to a target disease, for example, the severity of its symptom by the administration of the composition according to the present invention.

In addition, the present invention provides a food composition for preventing or alleviating dry eye syndrome or meibomian gland dysfunction, which includes 1,5-0-dicaffeoylquinic acid or a sitologically acceptable salt thereof as an active ingredient. The term "sitologically acceptable salt" includes salts derived from organic acids, inorganic acids, or bases. The food composition includes a health functional food composition.

When the 1,5-O-dicaffeoylquinic acid of the present invention or a sitologically acceptable salt thereof is used as a food additive, the compound may be added alone, or used in combination with other food or food ingredients, and may be suitably used according to a conventional method. The mixing amount of the active ingredient may be appropriately determined depending on the purpose of use (prevention, health, or therapeutic treatment). Generally, when manufacturing food or beverages, the 1,5-O-dicaffeoylquinic acid of the present invention or a sitologically acceptable salt thereof may be added at 15 wt% or less, or 10 wt% or less with respect to the raw materials. However, in the case of long-term ingestion for health and hygiene or health control, the amount may be less than the above range, and since there is no problem in terms of safety, the active ingredient can be used in amounts exceeding the above range.

There is no particular limitation on the type of food. Examples of food to which the above materials can be added include meat, sausages, bread, chocolates, candies, snacks, confectioneries, pizza, ramen, other noodles, gums, dairy products including ice cream, various kinds of soup, beverages, tea, drinks, alcohol beverages, and vitamin complexes, and includes all types of health functional food in the usual sense.

A health drink composition according to the present invention may contain various flavoring agents or natural carbohydrates as additional ingredients, like ordinary beverages. The above-mentioned natural carbohydrates are monosaccharides such as glucose and fructose, disaccharides such as maltose and sucrose, polysaccharides such as dextrin and cyclodextrin, and sugar alcohols such as xylitol, sorbitol, and erythritol. As a sweetener, natural sweeteners such as thaumatin and stevia extract or synthetic sweeteners such as saccharin and aspartame may be used. The proportion of natural carbohydrates is generally about 0.01 to 0.20g, or about 0.04 to 0.10g per 100 mL of the composition of the present invention.

In addition to the above, the composition of the present invention may contain various nutrients, vitamins, electrolytes, flavors, colorants, pectin acid and its salts, alginic acid and its salts, organic acids, protective colloidal thickeners, pH adjusters, stabilizers, preservatives, glycerin, alcohols or carbonating agents used in carbonated drinks. In addition, the composition of the present invention may contain pulp for the production of natural fruit juice, fruit juice drinks, and vegetable drinks. These ingredients may be used independently or in combination. The proportion of such additives is not very important, but is generally selected in in the range of 0.01 to 0.20 parts by weight per 100 parts by weight of the composition of the present invention.

The "health functional food" used herein is the same term as food for special health use (FoSHU), and refers to food with a high medicinal and healthcare effect that is processed to efficiently exhibit bioregulatory functions in addition to supplying nutrients. To obtain useful effects for preventing or ameliorating dry eye syndrome and/or meibomian gland dysfunction, the food may be prepared in various forms such as tablets, capsules, powder, granules, liquids, and pills.

The health functional food of the present invention may be produced by a method conventionally used in the art and may be produced by adding ingredients and components conventionally added in the art during production. In addition, unlike general drugs, it is made from food, so it has the advantage of not causing side effects that can occur with long-term drug use and is highly portable.

In addition, the present invention provides a quasi-drug for preventing or treating dry eye syndrome, which includes 1,5-O-dicaffeoylquinic acid or a pharmaceutically acceptable salt thereof as an active ingredient.

The "quasi-drug" used herein is a product used for treating, alleviating, or preventing a human or animal disease, and refers to a product which has a slight effect on the human body or does not directly act on the human body.

In addition to the above ingredients, the quasi-drug composition of the present invention may further include a pharmaceutically acceptable carrier, excipient, or diluent as needed. The pharmaceutically acceptable carrier, excipient or diluent is not limited as long as it does not affect the effect of the present invention, and may include, for example, a filler, an extender, a binder, a wetting agent, a disintegrant, a surfactant, a lubricant, a sweetener, a flavoring agent, or a preservative.

Representative examples of pharmaceutically acceptable carriers, excipients, or diluents used in the quasi-drug composition of the present invention may be lactose, dextrose, sucrose, sorbitol, mannitol, xylitol, maltitol, starch, gelatin, glycerin, acacia gum, alginate, calcium phosphate, calcium carbonate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, propylene glycol, polyethylene glycol, vegetable oil, injectable ester, Witepsol, Macrogol, Tween 61, cacao butter, or laurin butter.

When the composition of the present invention is provided as an external preparation, it may be formulated, but is not limited to, in the form of a solution, ointment, patch, gel, cream, or spray. According to one embodiment of the present invention, the quasi-drug of the present invention may include oral care products, including toothpastes, mouthwashes, and mouth sprays, ointments, masks, poultices, patches, and transdermal absorbents. The formulation method, dosage, usage method, or components of the quasi-drug may be appropriately selected from conventional techniques known in the technical field.

In addition, the present invention provides an ophthalmic composition, which includes 1,5-O-dicaffeoylquinic acid or a pharmaceutically acceptable salt thereof as an active ingredient.

The ophthalmic composition according to the present invention may include an additive such as a pharmaceutically acceptable carrier, excipient or diluent, and characteristics to be considered in excipients include, but are not limited to, compatibility with cyclosporine and trehalose, biocompatibility, a processing temperature, etc.

The ophthalmic composition according to the present invention may be prepared as a parenteral preparation such as an ophthalmic solution, an eye ointment, an injection, or an eyewash, or an oral preparation such as a tablet, a capsule, or a granule, and a preferred dosage form is an ophthalmic solution, and the most preferred dosage form is an eye drop.

When the ophthalmic composition according to the present invention is prepared in the form of a solution, it may be provided in any dosage form used as an ophthalmic solution, for example, an aqueous ophthalmic solution, such as an aqueous emulsion ophthalmic solution, a viscous ophthalmic solution, or a dissolved ophthalmic solution; or a non-aqueous ophthalmic solution such as a non-aqueous emulsion ophthalmic solution.

When the ophthalmic composition according to the present invention is prepared as an aqueous emulsion ophthalmic solution, it may include various additives known in the art as long as they do not impair the purpose of the present invention, and the additives may include, for example, an isotonic agent, a buffer, a stabilizer, a pH adjuster, a thickener, a preservative, a chelating agent, a solubilizer, and a solvent. The buffer may be selected from the group consisting of a phosphate buffer, a borate buffer, a citrate buffer, a tartrate buffer, an acetate buffer (e.g., sodium acetate) tromethamine, and amino acids, but the present invention is not limited thereto. Preferably, a phosphate buffer may be used. The isotonic agent may be selected from the group consisting of saccharides such as sorbitol, glucose, erythritol, and mannitol, polyhydric alcohols such as glycerin, polyethylene glycol, and polypropylene glycol, and sodium chloride, but the present invention is not limited thereto. The preservative may be selected from the group consisting of benzalkonium chloride, benzethonium chloride, alkyl paraoxybenzoates such as methyl paraoxybenzoate and ethyl paraoxybenzoate, benzyl alcohol, phenethyl alcohol, sorbic acid and its salts, thimerosal, polyquaternium, benzododecinium bromide, oxychloro complex, and chlorobutanol, but the present invention is not limited thereto. The stabilizer may be selected from the group consisting of water-soluble polymers such as cyclodextrin and derivatives thereof, and poly(vinylpyrrolidone), and surfactants such as polysorbate 80 (Tween 80), polysorbate 20, and tyloxapol, but the present invention is not limited thereto. The pH adjuster may be selected from the group consisting of hydrochloric acid, acetic acid, phosphoric acid, sulfuric acid, sodium hydroxide, potassium hydroxide, monoethaneolamine, aqueous ammonia, and ammonium hydroxide, but the present invention is not limited thereto. The thickener may be selected from the group consisting of hydroxyethylcellulose, hydroxypropylcellulose, methylcellulose, hydroxypropylmethylcellulose and carboxymethylcellulose, polyvinyl alcohol, carbomer, povidone, poloxamer, polycarbophil, and its salts, but the present invention is not limited thereto. The chelating agent may be selected from the group consisting of sodium edetate, sodium citrate, and condensed sodium phosphate, but the present invention is not limited thereto. The solubilizer or solvent may be selected from glycerin, DMSO, DMA, N-methylpyrrolidone, ethanol, benzyl alcohol, isopropyl alcohol, polyethylene glycol or propylene glycol of various molecular weights, but the present invention is not limited thereto. Since there may be some overlap between components that can be used as a solvent or solubilizer, and any component can be used as either a solvent or a solubilizer, when any component acts as a solvent in the preparation, it may be considered a solvent and when any component does not act as a solvent, it may be considered a solubilizer. Alternatively, a solubilizer may be a surfactant in some modifications. Surfactant combinations of various types of surfactants may be commercially used. For example, a non-ionic, anionic (i.e., soap or a sulfonate), cationic

(i.e., CTAB), zwitterionic, polymeric, or amphoteric surfactant may be used. For example, a surfactant that can be used includes, but is not limited to, one having an HLB of 10, 11, 12, 13, or 14 or higher. Examples of the surfactant include a polyoxyethylene product of hydrogenated vegetable oil, polyethoxylated castor oil or polyethoxylated hydrogenated castor oil, polyoxyl castor oil or derivatives thereof, polyoxyethylene-sorbitan-fatty acid ester, and polyoxyethylene castor oil derivatives, but the present invention is not limited thereto. According to an exemplary embodiment, the ophthalmic composition of the present invention may consist of 0.01 to 0.1 wt% of cyclosporine, 0.5 to 7.5 wt% of trehalose, 1 to 10 wt% of a solubilizer, 0.01 to 2 wt% of a solvent, and the remainder of a buffer and an isotonic agent based on the total weight of the composition.

The aqueous emulsion ophthalmic solution is preferably prepared in a nanoemulsion type, and in this case, various additives known in the art may be included as long as they do not impair the purpose of the present invention, and include, for example, an oil and a surfactant. The oil may be one or more selected from the group consisting of propylene glycol monocaprylate, propylene glycol laurate, medium chain (C8 - C10) triglycerides, glyceryl-1,3-dioleate, glyceryl monooleate, and glyceryl linoleate. The surfactant may be one or more selected from the group consisting of oleoyl macrogolglycerides, linoleoyl macrogolglycerides, caprylocaproyl polyoxylglycerides, polyoxyl 35 castor oil, polyoxyl 35 hydrogenated castor oil, polyoxyl 40 hydrogenated castor oil, polyoxyl 40 hydrogenated castor oil, a condensation product of ethylene oxide with 12-hydroxystearic acid, and polysorbate 80 (Croda, UK).

Most preferably, the aqueous emulsion ophthalmic solution may be prepared as transparent nanoemulsion-type eye drops, and to prepare this, reference may be made to, for example, Korean Patent Registration No. 10-0938500, but the present invention is not limited thereto.

The ophthalmic composition according to the present invention may be used for alleviating, preventing or treating an eye disease, and is preferably used for alleviating, preventing, or treating dry eye syndrome and/or meibomian gland dysfunction, and also used for alleviating, preventing or treating disorders caused by dry eyes. In addition, the ophthalmic composition according to the present invention may be used as a composition for promoting lacrimal secretion.

The ophthalmic composition according to the present invention may be provided by being filled in a sterilized container, may include instructions for its use, and the instructions may be physically attached to a first container filled with the ophthalmic composition, or a second container packaging the first container or packaged together inside the second container.

In addition, the ophthalmic composition according to the present invention includes an eye drop composition (composition for ocular instillation) and an artificial tear composition.

The artificial tear composition of the present invention may further include an electrolyte, a nonionic surfactant, an antibacterial agent, a borate-and-polyol complex, or a low molecular weight amino acid.

The electrolyte included in the artificial tear composition is used to stimulate natural tears, and includes ions included in natural tears as an ingredient. For example, the electrolyte used in the present invention is potassium, calcium, magnesium, or zinc, and its concentration is disclosed in U.S. Pat. No. 5,403,598.

The nonionic surfactant used in the composition of the present invention reduces the surface tension of the composition of the present invention and allows the solution to spread evenly on the corneal surface. Nonionic surfactants include various surfactants known in the art, but are preferably polysolvates such as polysolvate 80 (Tween 80) with an HLB value of 15 or more; and a block copolymer prepared by adding ethylene oxide and propylene oxide to an ethylenediamine. The amount of surfactant used in the present invention is meant to be sufficient to provide a composition having a surface tension of about 38 to 45 dynes/cm, and the above surface tension range corresponds to that of natural tears.

The artificial tear composition of the present invention may be prepared to a single-dose or multi-dose product. In order to maintain the sterility of the composition after commercialization, an antibacterial preservative is not necessary for a single-dose product, but may be necessary for a multi-dose product.

The artificial tear composition of the present invention is prepared to have a pH and osmotic pressure, which is compatible with the eye. Preferably, the pH is 6.8 to 7.8, and the osmotic pressure is 250 to 350 mOsm/kg. The osmotic pressure-adjusting material may be one or more selected from the group consisting of D-sorbitol, glycerin, concentrated glycerin, mannitol and maltitol syrups, and an ionic material, and when one type of isotonic agent is used, its composition ratio is about 0.5 to 20% by weight with respect to volume, and preferably, 1 to 10%, but the present invention is not limited thereto.

In order to increase the comfort and retention time of the composition when applying it to the eyes, the artificial tear composition of the present invention is prepared to have improved viscosity. Preferably, the viscosity may be 1 to 20 cps, more preferably, 2 to 20 cps, and most preferably, 5 to 20 cps.

The artificial tear composition of the present invention may be topically applied to the cornea for the prevention or treatment of dry eye syndrome and/or meibomian gland dysfunction, and may be used as an eye wetting agent or eye lubricant and applied by dropping one or two drops on the cornea.

In addition, the composition according to the present invention provides an ophthalmic kit. The ophthalmic kit includes a kit for preventing or alleviating dry eye syndrome or meibomian gland dysfunction.

The kit may be a pharmaceutical kit including the composition according to the present invention and a container that contains the composition. The container may include a dispensing means which is adapted to enable topical administration of the composition according to the present invention to a patient's eyes. For example, the container including the dispensing means may be a conventional dropper bottle such as a bottle manufactured with glass or a thermoplastic elastomer with an appropriate dispensing means or disposable dropper. In addition, the dispensing means includes a dropper with a dimension that dispenses droplets having a volume of about 8 to 15 µL, preferably about 8 to 12 µL, and more preferably about 10 µL. With a small droplet volume, accurate dosing to the eyes (avoiding over-dosing) may be achieved, and after administration, an excessive secretion of a substantial fraction of the composition from the eyes may be avoided.

The kit may further include instructions for use (e.g., manuals). The instructions for use may include information on the dosage, administration method, storage environment, the order of use, etc. of the composition according to the present invention. The instructions preferably include instructions for the use of the composition in the treatment, prevention, or alleviation of an eye disease, preferably, in the therapy of dry eye syndrome or meibomian gland dysfunction. The kit may be provided with instructions or a manual for use of the composition of the present invention in any appropriate form, for example, in the form of a printed or readable enclosed label or instruction leaflet, or on any suitable data carrier. Alternatively, the instructions may be provided in an electronic or computer-readable form such as a barcode or QR code.

In addition, the present invention provides a method of treating dry eye syndrome and/or meibomian gland dysfunction, which includes administering an effective amount of 1,5-O-dicaffeoylquinic acid, an isomer thereof, a solvate thereof, or a pharmaceutically acceptable salt thereof, or a composition including the same as an active ingredient to a subject in need thereof.

In addition, the present invention provides a use of 1,5-O-dicaffeoylquinic acid, an isomer thereof, a solvate thereof, or a pharmaceutically acceptable salt thereof, or a composition including the same as an active ingredient for preventing, alleviating or treating dry eye syndrome and/or meibomian gland dysfunction.

In addition, the present invention provides a use of 1,5-O-dicaffeoylquinic acid, an isomer thereof, a solvate thereof, or a pharmaceutically acceptable salt thereof, or a composition including the same as an active ingredient for preparing a drug for treating dry eye syndrome or meibomian gland dysfunction.

Hereinafter, preferred examples are presented to enable the present invention to be better understood. However, the following examples are merely provided to more easily understand the present invention, and the content of the present invention is not limited by the following examples.

### [Examples]

### Example 1. Confirmation of therapeutic effect of 1,5-O-dicaffeoylquinic acid (1,5DQ) in eye dry syndrome mouse model

Two types of dry eye syndrome mouse models were used to examine whether 1,5DQ instillation is effective for dry eye syndrome. The first mouse model is a desiccating stress model, and BALB/c mice were reared in a dry environment (humidity: 20 to 30%) while administering a drug that suppresses tear secretion (scopolamine hydrobromide 0.5 mg/0.2 mL) intraperitoneally three times a day. The second mouse model is a Sjögren's syndrome-related inflammatory dry eye syndrome mouse model, and NOD.B10.*H2*^{b} mice were used. In the NOD.B10.*H2*^{b} mice, under normal environments, inflammation in the lacrimal glands and on the ocular surface spontaneously increases at about 11 weeks, leading to destruction of the lacrimal glands and ocular surface, decreased tear secretion, and severe dry eye syndrome accompanied by ocular surface epithelial defects progresses between 11 and 14 weeks.

In each of the two types of dry eye syndrome mouse models, the dry eye treatment effect of 1,5DQ instillation was investigated. The degree of corneal epithelial defects was measured by instilling a drop of the Lissamine green vital dye on the cornea and then observing the degree of stained corneal epithelial defects under a microscope and clinical scoring the same. The amount of tear secretion was evaluated by placing a thread soaked with a phenol red dye on the outer canthus of the mouse for 1 minute and measuring the length moistened by tears (length of the dyed area).

### 1-1. 1,5DQ reduces corneal epithelial defects and increases amount of tear secretion in a dry eye syndrome mouse model induced by desiccating stress.

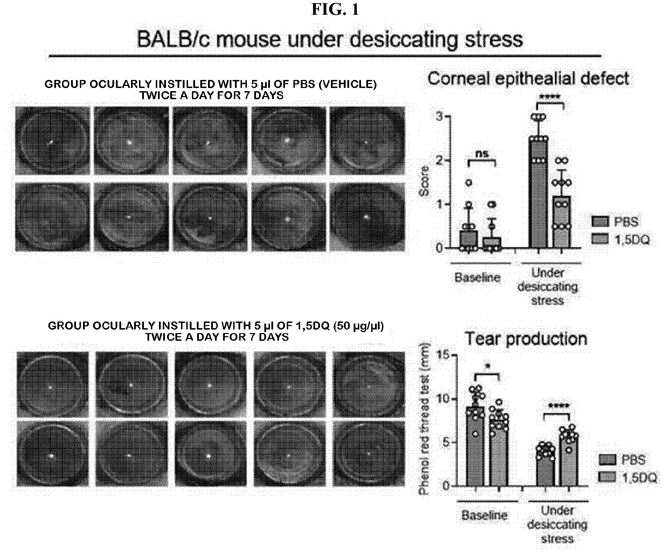

8-week-old BALB/c mice undergoing desiccating stress were divided into two groups, and (i) 5 µL of 1,5DQ (50 µg/µL) or (ii) 5 µL of PBS was instilled into both eyes of each mouse twice a day for 5 days, and then the degree of corneal epithelial defects and the amount of tear secretion were evaluated. As a result, it was confirmed that, compared to the PBS-instilled positive control, the 1,5DQ-instilled treatment group exhibited significantly reduced corneal epithelial defects. In addition, it was confirmed that, when undergoing desiccating stress, the amount of tear secretion significantly increased in the 1,5DQ-instilled experimental group compared to the PBS-instilled positive control (FIG. 1).

### 1-2. 1,5DQ reduces corneal epithelial defects and increases amount of tear secretion in a Sjögren's syndrome-related dry eye mouse model.

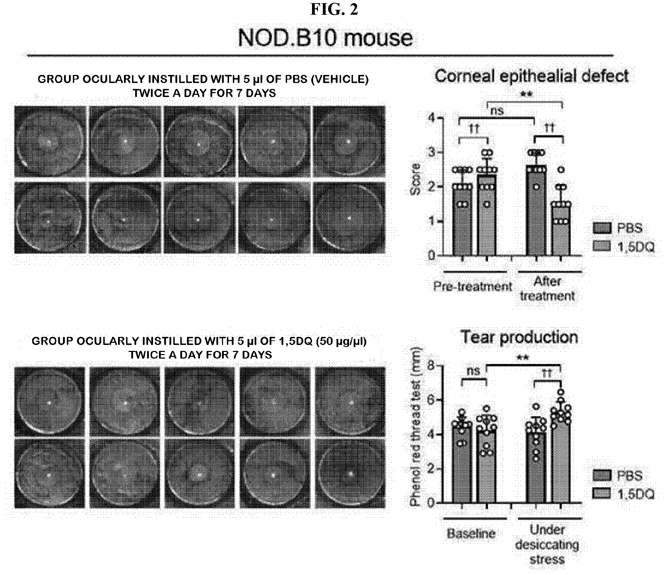

As described above, as Sjögren's syndrome-related inflammatory dry eye syndrome mice, NOD.B10.*H2*^{b} mice were used. 12-week-old mice were divided into two groups, and (i) 5 µL of 1,5DQ (50 µg/µL) or (ii) 5 µL of PBS was instilled into both eyes of each mouse twice a day for 5 days, and then the degree of corneal epithelial defects and the amount of tear secretion were evaluated. As a result, compared to the PBS-instilled positive control, the 1,5DQ-instilled treatment group exhibited significantly reduced corneal epithelial defects. In addition, it was confirmed that, when undergoing desiccating stress, the amount of tear secretion significantly increased in the 1,5DQ-instilled experimental group compared to the PBS-instilled positive control (FIG. 2).

The above experimental results show that the compound according to the present invention can alleviate or treat dry eye syndrome by reducing corneal epithelial defects, which are the main causes of dry eye syndrome, and promoting tear secretion.

### Example 2. Identification of dry eye syndrome treatment mechanism of 1,5DQ in dry eye syndrome mouse model: anti-inflammatory action on lacrimal grand

### 2-1. 1,5DQ suppresses inflammatory cytokine IL-1β expression in the lacrimal glands of a dry eye syndrome mouse model.

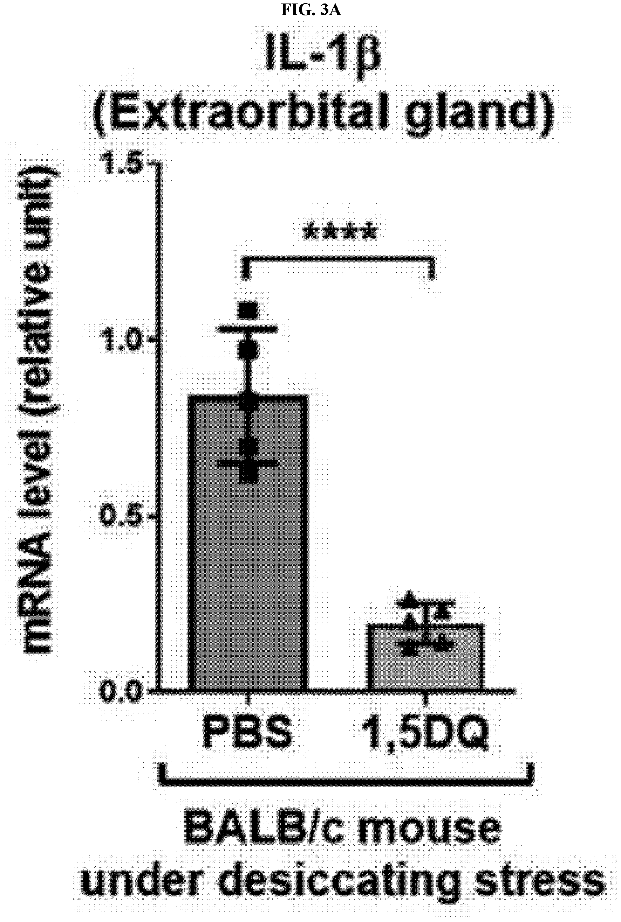

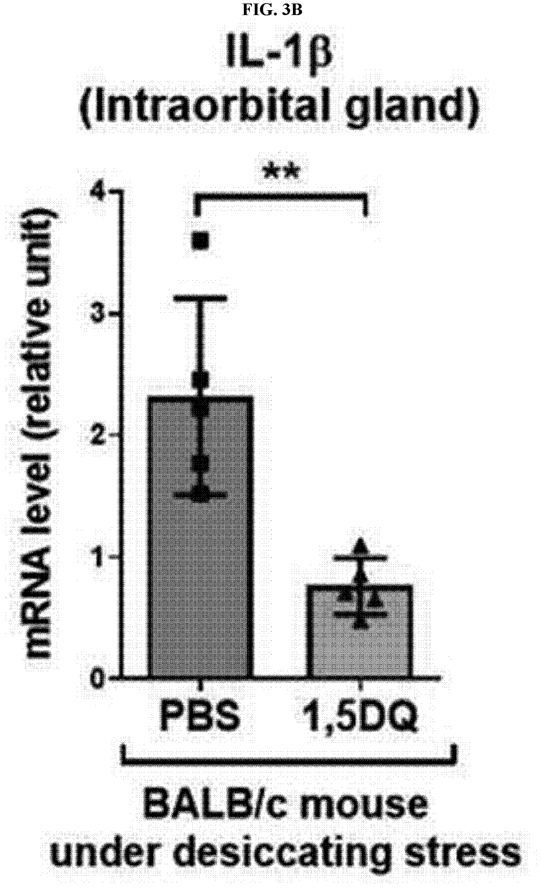

Desiccating stress-induced 8-week-old BALB/c mice were divided into two groups, and (i) 5 µL of 1,5DQ (50 µg/µL) or (ii) 5 µL of PBS was instilled into both eyes of each mouse twice a day for 5 days. The lacrimal glands of a mouse were classified into an extraorbital lacrimal gland and an intraorbital lacrimal gland, and the degree of inflammatory activation in each lacrimal gland was evaluated by measuring the levels of transcripts of inflammatory cytokine interleukin 1β (IL-1β) using real-time RT PCR. As a result, it was confirmed that interleukin 1β expression was significant reduced in both the extraorbital and intraorbital lacrimal glands in the 1,5DQ-instilled group compared to the PBS-administered positive control (FIGS. 3A and 3B).

### 2-2. 1,5DQ improve lacrimal gland function by reducing the infiltration of CD3 T inflammatory cells in lacrimal glands in a Sjögren's syndrome-related inflammatory dry eye mouse model.

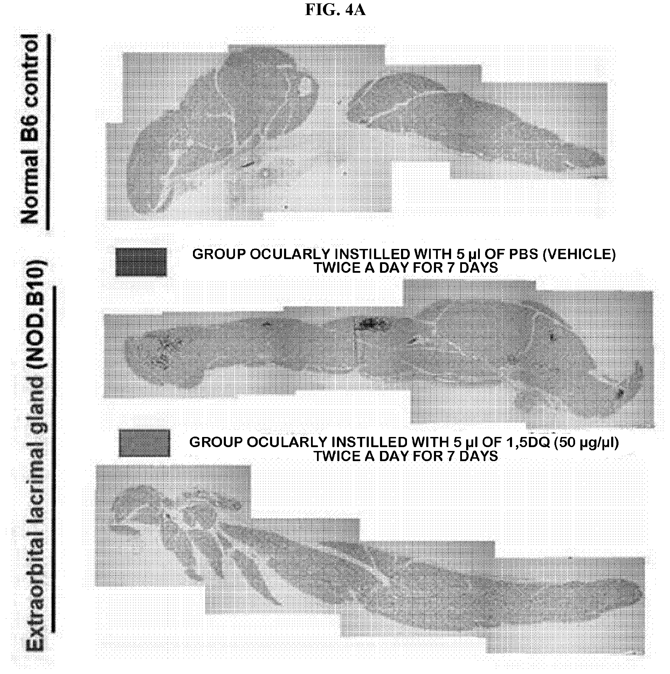

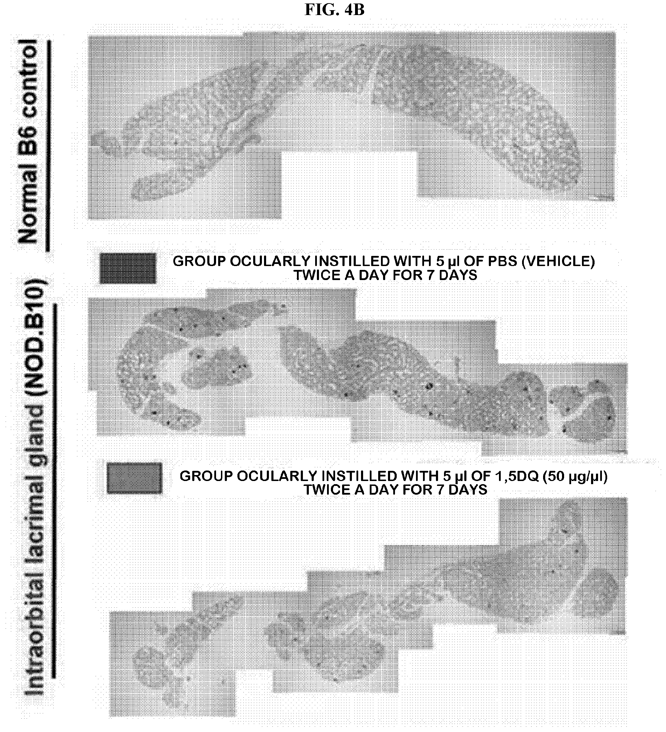

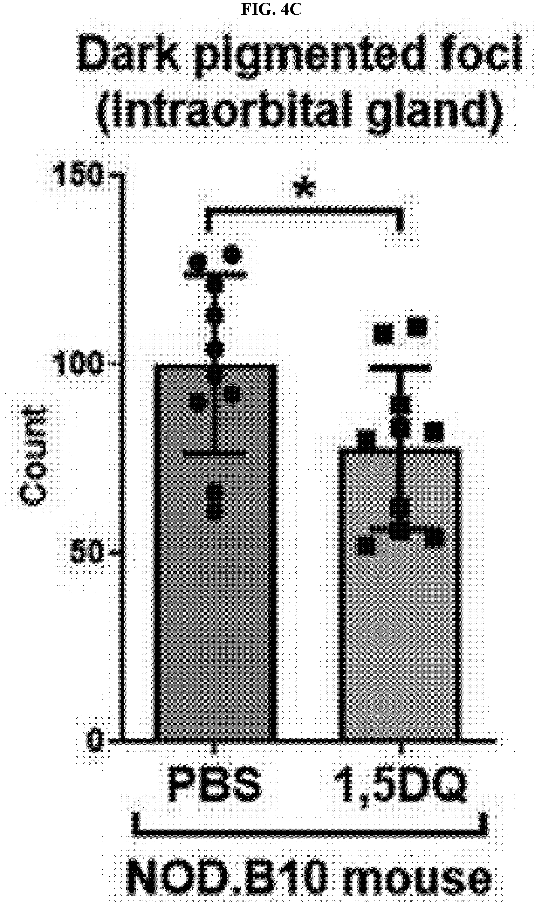

Sjögren's syndrome-related inflammatory dry eye syndrome mice, i.e., NOD.B10.*H2*^{b} mice, typically show increased infiltration of inflammatory cells into the lacrimal glands between 11 to 14 weeks old. 12-week-old NOD.B10.*H2*^{b} mice were divided into two groups, and (i) 5 µL of 1,5DQ (50 µg/µL) or (ii) 5 µL of PBS was instilled into both eyes of each mouse twice a day for 5 days, and then the level of inflammatory cell infiltration in the lacrimal glands was evaluated. In addition, the extraorbital and intraorbital lacrimal glands, which had been removed from each mouse and sectioned, were immunostained with CD3, T cells were observed using the stained CD3 under a microscope (FIGS. 4A and 4B) and counted, and then the level of immune cell infiltration in the lacrimal glands was quantified (FIG. 4C). As a result, it was confirmed that the number of CD3-positive immune cells deposited in the lacrimal glands significantly decreased in the 1,5DQ-instilled group compared to the PBS-instilled positive control.

The above experimental results show that the compound according to the present invention can exert an excellent anti-inflammatory effect on the lacrimal gland, thereby improving lacrimal gland function and treating dry eye syndrome.

### Example 3. Identification of dry eye syndrome treatment mechanism of 1,5DQ in dry eye syndrome mouse model: improvement in meibomian gland function

The tear film is composed of a mucus layer, an aqueous layer, and a lipid layer, tears in the aqueous layer are secreted from the lacrimal glands, and tears in the lipid layer are secreted from the meibomian glands present in the eyelids. Therefore, using dry eye syndrome mouse models, the effect of1,5DQ instillation on the improvement in meibomian gland function was investigated.

### 3-1. 1,5DQ suppresses inflammation of eyelid meibomian glands in a Sjögren's syndrome-related inflammatory dry eye mouse model.

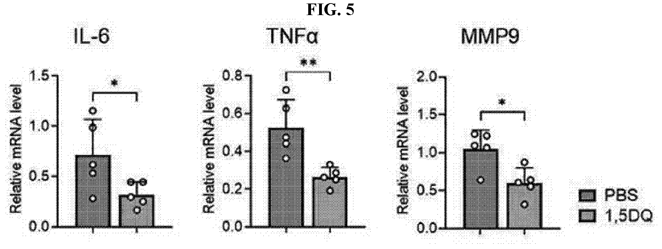

12-week-old NOD.B10.*H2*^{b} mice were divided into two groups, and (i) 5 µL of 1,5DQ (50 µg/µL) or (ii) 5 µL of PBS was instilled into both eyes of each mouse twice a day for 5 days. The degree of inflammation activation in the eyelids was evaluated by measuring the levels of transcripts of the inflammatory cytokines IL-6 and tumor necrosis factor-α (TNF-α), and another inflammatory factor, matrix metallopeptidase 9 (MMP-9), using real-time RT PCR. As a result, it was confirmed that the IL-6, TNF-α, and MMP-9 expression in the lacrimal glands (meibomian glands) of the eyelids was significantly reduced in the 1,5DQ instillation group compared to the PBS-administered positive control (FIG. 5).

The above results show that the compound according to the present invention can exert an anti-inflammatory effect in the meibomian glands of the eyelids and can treat or alleviate dry eye syndrome.

### 3-2. 1,5DQ increases the area of eyelid meibomian glands in a Sjögren's syndrome-related inflammatory dry eye mouse model.

When the area of the meibomian glands decreases, the lipid layer of the tear film becomes thinner, which can cause or aggravate dry eye syndrome. Therefore, the area of the meibomian glands is a key judgment factor in diagnosing meibomian gland dysfunction.

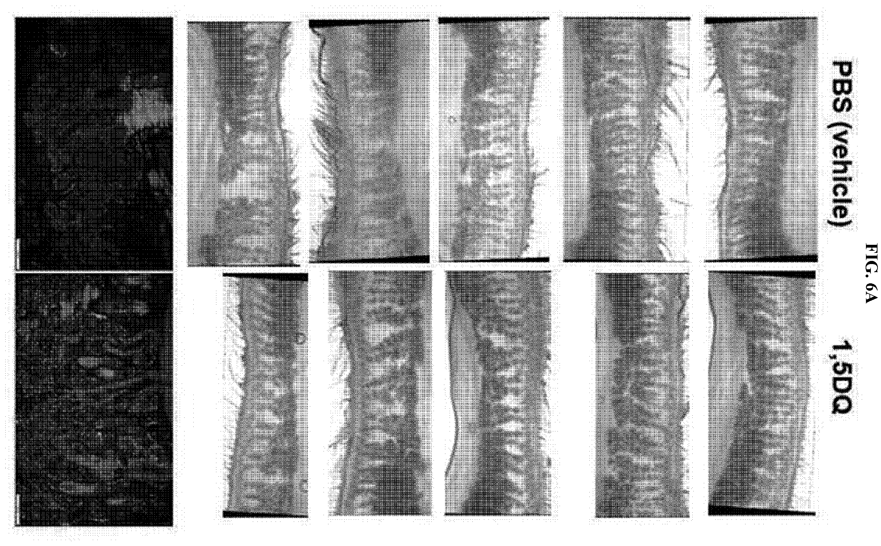

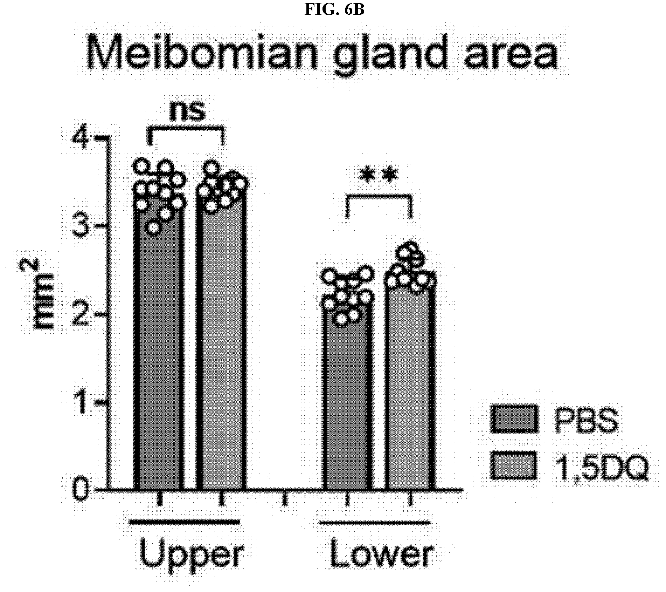

12-week-old NOD.B10.*H2*^{b} mice were divided into two groups, and (i) 5 µL of 1,5DQ (50 µg/µL) or (ii) 5 µL of PBS was instilled into both eyes of each mouse twice a day for 5 days. The area of the meibomian glands was confirmed by removing an eyelid from the mouse model and then measuring the area of 3 mm at the center. As a result, it was confirmed that the area of the meibomian glands in the lower eyelid significantly increased in the 1,5DQ instillation group compared to the PBS-administered positive control (FIGS. 6A and 6B).

The above results show that the compound according to the present invention increases the area of meibomian glands, and can improve the function of the meibomian glands.

### 3-3. 1,5DQ increases transcription of meibomian gland differentiation factors of eyelids in a desiccating stress-induced dry eye syndrome mouse model.

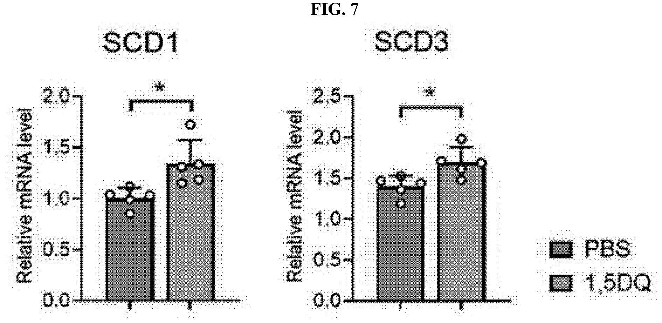

12-week-old NOD.B10.*H2*^{b} mice were divided into two groups, and (i) 5 µL of 1,5DQ (50 µg/µL) or (ii) 5 µL of PBS was instilled into both eyes of each mouse twice a day for 5 days. After removing the lower and upper eyelids of each mouse model, the expression level of meibomian gland differentiation factors in the eyelids was determined using real-time RT PCR. The meibomian glands are composed of sebocytes, and the transcription levels of stearoyl-CoA desaturase (SCD)1 and SCD3, which are sebocyte-specific genes, were measured. As a result, it was confirmed that the expression of SCD1 and SCD3 in the eyelids significantly increased in the 1,5DQ instillation group compared to the PBS administration group (FIG. 7).

The above results show that the compound according to the present invention improves the function of the meibomian glands by promoting the expression of meibomian gland differentiation factors.

Taken together, the above experimental results demonstrate that the compound according to the present invention suppresses inflammation of the meibomian glands and improves the function of the meibomian glands, resulting in an effect of improving and treating dry eye syndrome.

### Example 4. Confirmation of effect of treating dry eye syndrome according to 1,5DQ treatment concentration in dry eye syndrome mouse model

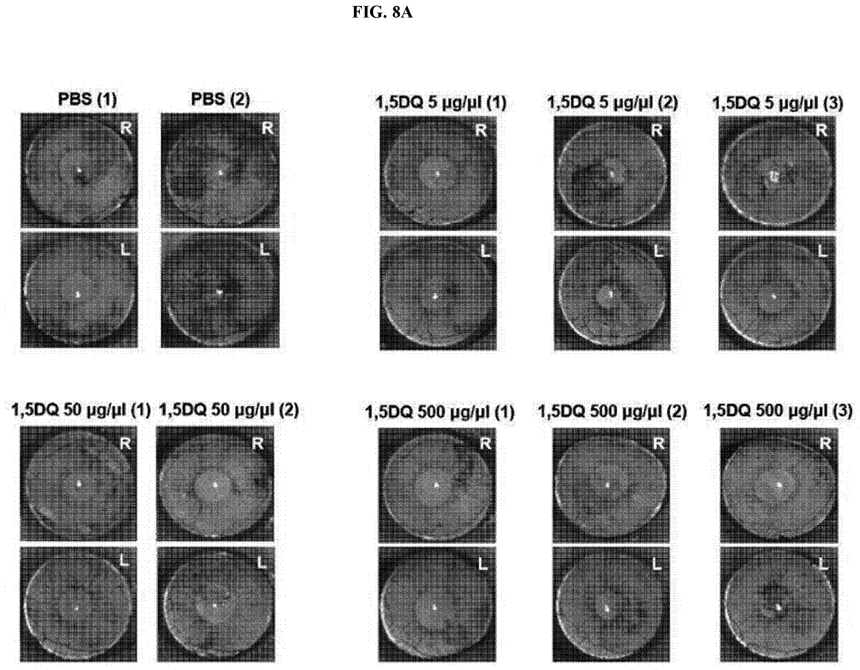

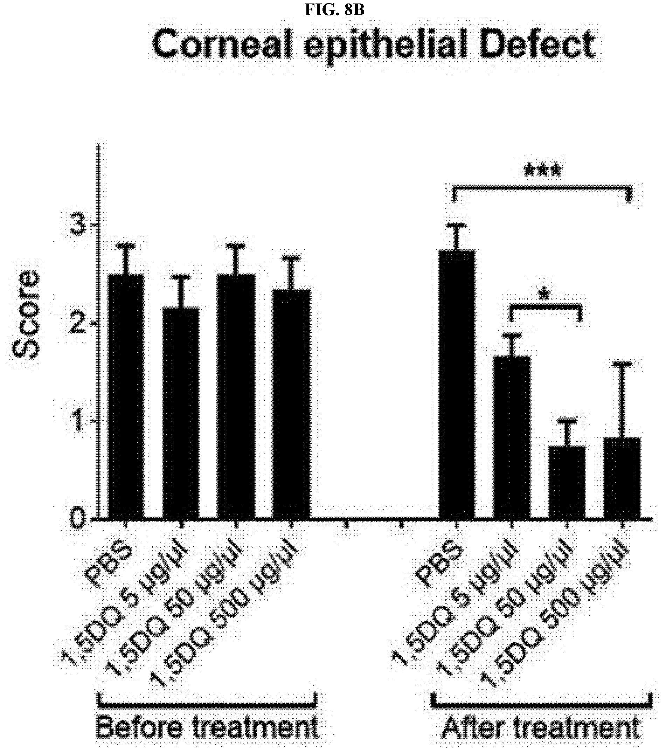

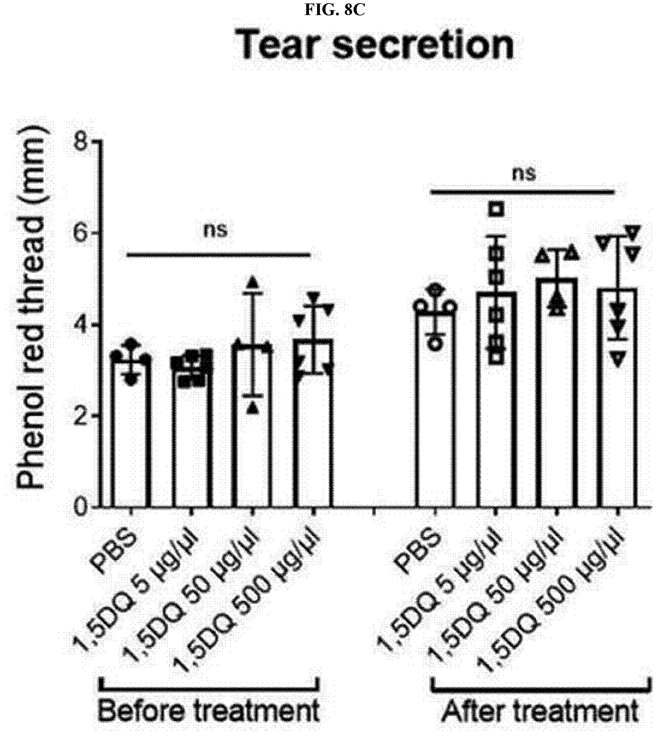

Through the previous example, it was confirmed that the administration of 5 µL of 1,5DQ (50 µg/µL) twice a day had a significant dry eye syndrome treatment effect in a dry eye syndrome mouse model. Accordingly, the dry eye syndrome treatment effect according to the 1,5DQ treatment concentration was investigated. 12-week-old NOD.B10.*H2*^{b} mice (Sjögren's syndrome-related inflammatory dry eye syndrome mouse models) were divided into four groups, and (i) 5 µL of PBS, (ii) 5 µL of 1,5DQ (5 µg/µL), (iii) 5 µL of 1,5DQ (50 µg/µL), or (iv) 5 µL of 1,5DQ (500 µg/µL) was instilled into both eyes twice a day for 5 days, and then the degree of corneal epithelial defects and the amount of tear secretion were evaluated. As a result, it was confirmed that, compared to the groups administered PBS or a low concentration of 1,5DQ (5 µg/µL), corneal epithelial defects were significantly reduced in the groups in which a high concentration of 1,5DQ (50 µg/µL or 500 µg/µL) was instilled (FIGS. 8A and 8B). There was no statistically significant difference in the amount of tear secretion between the groups, but it showed an increasing trend depending on concentration (FIG. 8C).

The above results show that the dry eye syndrome treatment effect of the compound according to the present invention has a concentration-dependent pattern.

### Example 5. Identification of dry eye syndrome treatment mechanism of 1,5DQ in dry eye syndrome mouse model: Increase in survival of corneal epithelial cells and suppression of apoptosis thereof

An important cause of corneal epithelial cell damage due to dry eye syndrome is increased tear evaporation due to the instability of the tear film, which causes corneal epithelial cell damage and apoptosis due to hyperosmotic stress. Therefore, when human corneal epithelial cells are exposed to a hyperosmotic environment, which is a dry eye syndrome environment, it was confirmed through an in vitro experiment that cell viability increases or apoptosis decreases in response to 1,5DQ treatment.

### 5-1. 1,5DQ increases viability of corneal epithelial cells.

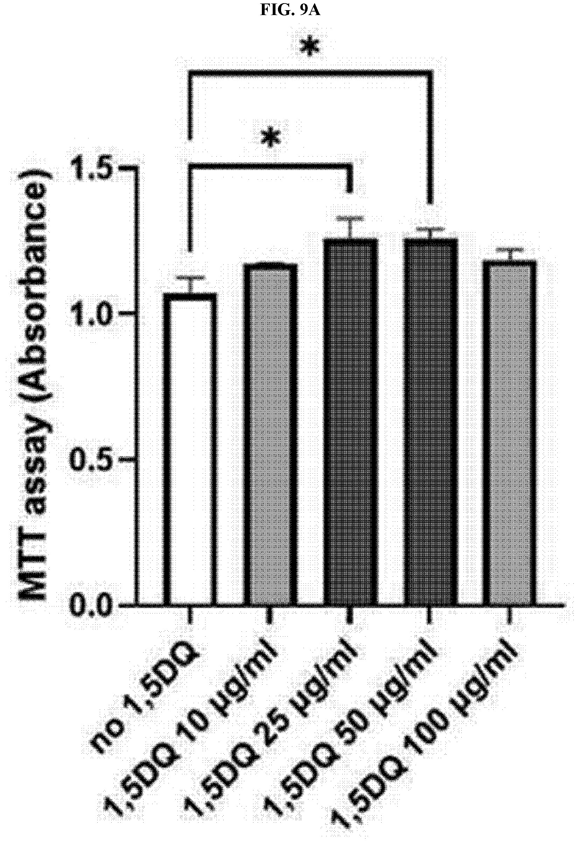

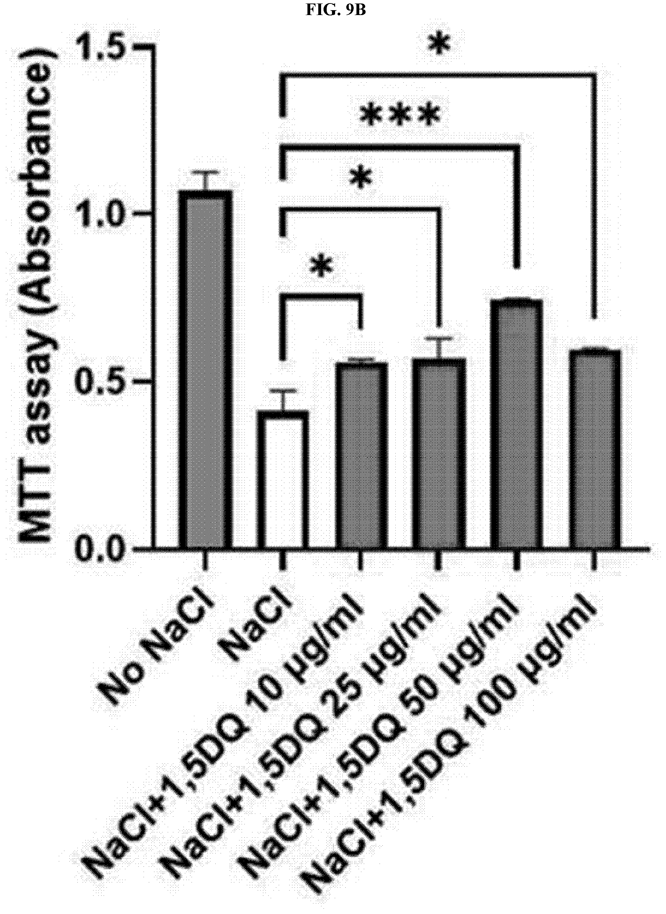

Corneal epithelial cells primarily cultured from human corneal limbal cells were treated with 100 mM NaCl for exposure to a hyperosmotic environment, and treated with 0, 10, 25, 50, or 100 µg/mL of 1,5DQ for 24 hours, and then cell viability was evaluated through MTT assay. As a result, in normal corneal epithelial cells that were not treated with NaCl, viability significantly increased by 1,5DQ treatment (25 µg/mL or 50 µg/mL) (FIG. 9A), and compared to an NaCl-untreated control, viability was greatly reduced in 100 mM NaCl-treated corneal epithelial cells, whereas viability significantly increased by treatment with 10, 25, 50, or 100 µg/mL of 1,5DQ (FIG. 9B).

The above results show that the compound according to the present invention increased the viability of corneal epithelial cells not only in a normal environment but also in a hyperosmotic environment that causes dry eye syndrome.

### 5-2. 1,5DQ suppresses the apoptosis of corneal epithelial cells.

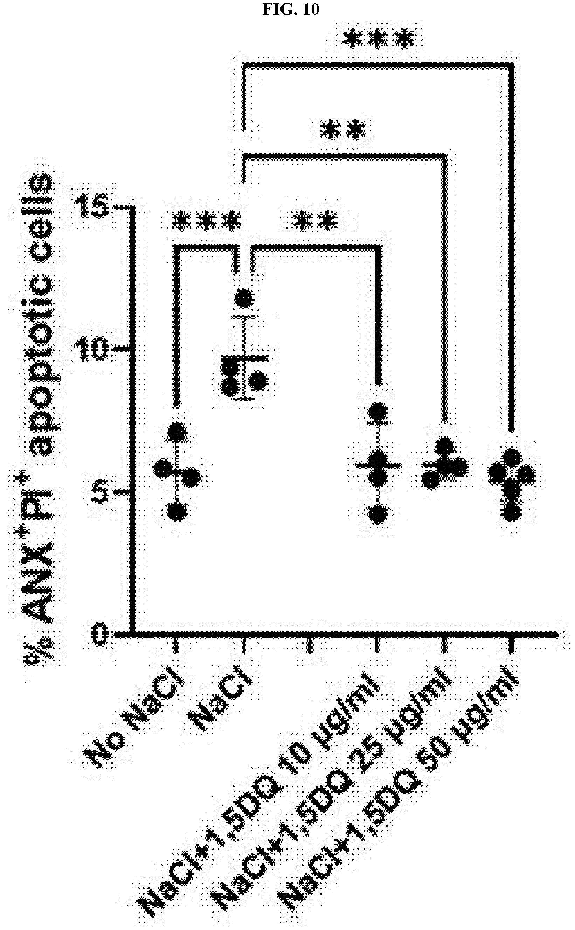

Corneal epithelial cells primarily cultured from human corneal limbal cells were treated with 100 mM NaCl for exposure to a hyperosmotic environment, and treated with 0, 10, 25, 50, or 100 µg/mL of 1,5DQ for 24 hours, and the degree of apoptosis was measured by performing flow cytometry after annexin V (ANX) and propidium iodide (PI) staining. As a result, it was confirmed that the treatment with 10, 25, and 50 µg/mL of 1,5DQ significantly reduced the apoptosis level of the corneal epithelial cells, which had increased due to NaCl treatment, and restored it to a normal level (FIG. 10).

The above results show that the compound according to the present invention can suppress the apoptosis of corneal epithelial cells in a hyperosmotic environment to prevent or treat dry eye syndrome.

As described above, it was confirmed that 1,5-O-dicaffeoylquinic acid not only increases the amount of tear secretion in an animal model with dry eye syndrome induced by desiccating stress or an autoimmune disease (Sjögren's syndrome), restores corneal epithelial defects, suppresses inflammation of the lacrimal glands and improves their function, but also promotes the differentiation, an increase in area, and the suppression of inflammation in the meibomian glands. Therefore, 1,5-O-dicaffeoylquinic acid can not only effectively treat dry eye syndrome caused by the environment or disease, but also improve meibomian gland function, so it is expected to be used as a treatment for various eye diseases.

It should be understood by those of ordinary skill in the art that the above description of the present invention is exemplary, and the exemplary embodiments disclosed herein can be easily modified into other specific forms without departing from the technical spirit or essential features of the present invention. Therefore, it should be interpreted that the exemplary embodiments described above are illustrative in all aspects, and not restrictive.

### [Industrial Applicability]

The present invention relates to a composition for preventing, alleviating, or treating dry eye syndrome or meibomian gland dysfunction, and it was confirmed that 1,5-0-dicaffeoylquinic acid not only reduces corneal epithelial defects and increases the amount of tear secretion, but also suppresses inflammation in the lacrimal glands and the meibomian glands, increases the area of the meibomian glands, increases the viability of corneal epithelial cells in a hyperosmotic environment, and suppresses apoptosis, thereby exhibiting an effect of preventing, alleviating, or treating dry eye syndrome or meibomian gland dysfunction. Therefore, the composition according to the present invention cannot only effectively treat dry eye syndrome caused by environmental stress or various diseases, but can also improve the function of the meibomian glands, so it is expected to be used as a treatment for various eye diseases.

## Claims

1. A pharmaceutical composition for preventing or treating dry eye syndrome or meibomian gland dysfunction, comprising 1,5-O-dicaffeoylquinic acid, an isomer thereof, a solvate thereof, or a pharmaceutically acceptable salt thereof as an active ingredient.

2. The pharmaceutical composition of claim 1, wherein the dry eye syndrome or meibomian gland dysfunction is caused by one or more selected from the group consisting of Sjögren's syndrome, styes, blepharitis, a dry environment, keratoconjunctival epithelial disorder, corneal xerosis, alacrima, ocular graft-versus-host disease, Stevens-Johnson syndrome, ocular pemphigoid, drug-induced dry eye syndrome, dry eye syndrome after ophthalmic surgery, dry eye syndrome with allergic conjunctivitis, visual display terminal (VDT) syndrome, and contact lens wear.

3. The pharmaceutical composition of claim 1, wherein the composition is a formation for oral administration or eye drops.

4. The pharmaceutical composition of claim 1, wherein the composition satisfies one or more selected from the group consisting of the following:
an increase in the amount of tear secretion in the lacrimal glands;
a decrease in corneal epithelial defects;
a decrease in the IL-1β expression level or activity in the lacrimal glands; and
a decrease in the infiltration of CD3-positive immune cells in the lacrimal glands.

5. The pharmaceutical composition of claim 1, wherein the composition satisfies one or more characteristics selected from the group consisting of the following:
(a) a decrease in the expression level or activity of one or more selected from the group consisting of IL-6, TNFα, and MMP9 in the meibomian glands;
(b) an increase in the area of meibomian glands; and
(c) an increase in the expression level or activity of meibomian gland differentiation factor in the eyelids.

6. The pharmaceutical composition of claim 1, wherein
the composition satisfies one or more characteristics selected from the group consisting of the following:
(a) increased survival of corneal epithelial cells in dry eye syndrome or similar stress environments; and
(b) decreased apoptosis of corneal epithelial cells in dry eye syndrome or similar stress environments.

7. A kit for preventing or treating dry eye syndrome or meibomian gland dysfunction, comprising the composition of claim 1.

8. A food composition for preventing or alleviating dry eye syndrome or meibomian gland dysfunction, comprising 1,5-O-dicaffeoylquinic acid, an isomer thereof, a solvate thereof, or a sitologically acceptable salt thereof as an active ingredient.

9. An ophthalmic composition comprising 1,5-O-dicaffeoylquinic acid, an isomer thereof, a solvate thereof, or a pharmaceutically acceptable salt thereof as an active ingredient.

10. A method of treating dry eye syndrome or meibomian gland dysfunction, comprising: administering a composition comprising 1,5-O-dicaffeoylquinic acid, an isomer thereof, a solvate thereof, or a pharmaceutically acceptable salt thereof as an active ingredient to a subject in need thereof.

11. A use of a composition comprising 1,5-O-dicaffeoylquinic acid, an isomer thereof, a solvate thereof, or a pharmaceutically acceptable salt thereof as an active ingredient for preventing, alleviating or treating dry eye syndrome or meibomian gland dysfunction.

12. A use of a composition comprising 1,5-O-dicaffeoylquinic acid, an isomer thereof, a solvate thereof, or a pharmaceutically acceptable salt thereof as an active ingredient for preparing a drug for treating dry eye syndrome or meibomian gland dysfunction.
